(19) Europäisches Patentamt
European Patent Office
Office européen des brevets

(11) **EP 2 108 356 B1**

(12) **EUROPÄISCHE PATENTSCHRIFT**

(45) Veröffentlichungstag und Bekanntmachung des Hinweises auf die Patenterteilung:
**25.01.2017 Patentblatt 2017/04**

(51) Int Cl.:
***A61K 6/083*** *(2006.01)*

(21) Anmeldenummer: **08007195.4**

(22) Anmeldetag: **11.04.2008**

(54) **Konditioniermittel für das Ätzen von Schmelzläsionen**

Conditioning agent for the etching of enamel lesions

Produit de conditionnement pour graver les lésions de l'émail dentaire

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(43) Veröffentlichungstag der Anmeldung:
**14.10.2009 Patentblatt 2009/42**

(73) Patentinhaber:
• **Mühlbauer Technology GmbH
22547 Hamburg (DE)**
• **Charité - Universitätsmedizin Berlin
10117 Berlin (DE)**

(72) Erfinder:
• **Neffgen, Stephan
22459 Hamburg (DE)**
• **Neander, Swen Dr.
20148 Hamburg (DE)**
• **Lübbers, Dierk Dr.
25469 Halstenbek (DE)**

(74) Vertreter: **Glawe, Delfs, Moll
Partnerschaft mbB
von Patent- und Rechtsanwälten
Postfach 13 03 91
20103 Hamburg (DE)**

(56) Entgegenhaltungen:
**WO-A-00/09030 WO-A-2007/131725
US-A- 4 348 381 US-A- 6 036 944**

• **MEYER-LUECKEL ET AL: "Influence of the application time on the penetration of different dental adhesives and a fissure sealant into artificial subsurface lesions in bovine enamel" DENTAL MATERIALS, ELSEVIER, Bd. 22, Nr. 1, 1. Januar 2006 (2006-01-01), Seiten 22-28, XP005214018 ISSN: 0109-5641**
• **MEYER-LUECKEL H ET AL: "SURFACE LAYER EROSION OF NATURAL CARIES LESIONS WITH PHOSPHORIC AND HYDROCHLORIC ACID GELS IN PREPARATION FOR RESIN INFILTRATION" CARIES RESEARCH, S. KARGER AG, BASEL, CH, Bd. 41, Nr. 3, 1. April 2007 (2007-04-01), Seiten 223-230, XP008078691 ISSN: 0008-6568**

**Beschreibung**

Die Erfindung betrifft ein Kit zur Infiltration von Schmelzläsionen, das eine Zusammensetzung zur Verwendung als Konditioniermittel für das Ätzen von Schmelzläsionen enthält.

Karies ist eine weitverbreitete Zivilisationskrankheit. Jede letztendlich zu einer Kavitation führende Karieserkrankung beginnt mit einer Demineralisierung des Zahnschmelzes und wird in diesem Stadium initiale Zahnschmelzkaries genannt. In diesem Stadium ist die Karies zunächst nicht oder lediglich als sogenannte weiße Zahnschmelzläsion sichtbar, führt aber zu einem porösen Bereich des Zahns unterhalb dessen Oberfläche.

Eine solche Schmelzläsion kann grundsätzlich remineralisiert oder mit einem Kunstharz infiltriert werden (WO 2007/131725 A1). Problematisch bei der Infiltration mit Kunstharz ist allerdings, dass typischerweise eine in den Zahnschmelz hineinreichende Läsion an der Zahnoberfläche eine sogenannte pseudointakte Oberflächenschicht aufweist, die einen höheren Mineralgehalt im Vergleich zu der tiefergehenden Schmelzläsion aufweist und die Penetrierung der Schmelzläsion mit dem zur Infiltration verwendeten Kunstharz, dem sogenannten Infiltranten, erschwert oder verhindert. Es ist daher bereits vorgeschlagen worden, diese pseudointakten Oberflächenschichten durch ein Ätzmittel vorzubehandeln, so dass nach diesem Konditionieren der Infiltrant besser in die Läsion einpenetrieren kann.

Der Erfindung liegt die Aufgabe zugrunde, ein wirksames und gut lagerfähiges und anwendbares Konditioniermittel für das Ätzen von Schmelzläsionen zu schaffen.

Die Erfindung betrifft somit einen Kit zur Durchführung einer Infiltration von Zahnschmelz, mit den Bestandteilen:

    a) einem Konditionierer für das Ätzen von Schmelzläsionen, der enthält:

        a2) 4 bis 80 Gew.-% wenigstens einer Säure, die einen pKs-Wert von 2 oder weniger aufweist;

        b2) ein protisches Lösungsmittel;
        wobei die Säure bei 20°C einen Sättigungsdampfdruck von 120 mbar oder weniger aufweist;

    b) einem Infiltranten.

Die Säure mit einem pKs-Wert von 2 oder weniger kann eine anorganische oder organische Säure sein, Mischungen mehrerer Säuren sind ebenfalls möglich. Soweit es sich um eine mehrbasige Säure handelt, muss die erste Dissoziationsstufe aus dem in der Zusammensetzung vorliegenden Zustand der Säure einen pKs-Wert von 2 oder weniger aufweisen. Bevorzugte Obergrenzen für den pKs-Wert sind 1,5 und 1.

Bei dem protischen Lösungsmittel kann es sich insbesondere um Alkohole oder Wasser bzw. Mischungen daraus handeln. Erfindungsgemäß ist vorgesehen, dass die Säure und/oder die komplette Zusammensetzung bei 20°C einen Sättigungsdampfdruck von 120 mbar oder weniger aufweisen. Der Sättigungsdampfdruck ist der Druck der gasförmigen Phase eines Stoffes, wenn Flüssig- und Gasphase sich im Gleichgewicht befinden.

Im Rahmen der Erfindung wird bevorzugt der Partialdampfdruck lediglich der Säure in der Zusammensetzung betrachtet, es kann dann der Dampfdruck des Lösungsmittels außer Acht gelassen werden.

Die Erfindung hat erkannt, dass im Stand der Technik (WO 2007/131725 A1) als Konditioniermittel verwendete Salzsäure eine Reihe von Nachteilen aufweist. Um als Konditioniermittel hinreichend wirksam zu sein, muss eine etwa 15%ige Salzsäure verwendet werden. HCl besitzt in höher konzentrierten wässrigen Lösungen einen erheblichen Dampfdruck, so dass es gasförmig aus der wässrigen Salzsäurelösung entweicht. Entsprechende Salzsäurelösungen sind also wenig lagerstabil; zudem bestehen wegen der hohen Aggressivität des gasförmig entweichenden HCl hohe Anforderungen an die Beständigkeit der Verpackung des Konditioniermittels. Ferner kann bei der Anwendung entweichendes gasförmiges HCl biologische Gewebe wie beispielsweise das Zahnfleisch schädigen.

Die Erfindung hat erkannt, dass die im Patentanspruch näher definierten Säuren mit einem bestimmten Mindestwert der Säurestärke die remineralisierten Oberflächenschichten natürlicher Schmelzläsionen wirksam erodieren und damit für das Einpenetrieren eines Infiltranten vorbereiten können. Solche pseudointakten Oberflächenschichten einer natürlichen Schmelzläsion sind typischerweise 10 bis 40 µm dick und werden durch eine erfindungsgemäße Zusammensetzung dennoch in handhabbaren Applikationszeiten (beispielsweise etwa 90 bis 120 s) weitgehend vollständig durchdrungen und entfernt.

Der erfindungsgemäß vorgesehene niedrige Sättigungsdampfdruck stellt sicher, dass es -anders als bei der Verwendung von Salzsäure- nicht zum Entweichen aggressiver Säurebestandteile bei Lagerung oder Anwendung der erfindungsgemäßen Zusammensetzung kommt.

Geeignete Säuren sind beispielsweise Fluorsulfonsäure, organische Sulfonsäuren wie bspw. Trifluormethansulfonsäure, Methansulfonsäure, Toluolsulfonsäure, Amidosulfonsäure, Benzolsulfonsäure, Perchlorsäure, Schwefelsäure, Salpetersäure, Trichloressigsäure, Trifluoressigsäure, Pikrinsäure oder Semiquadratsäure. Bevorzugte Säuren sind Methansulfonsäure und p-Toluolsulfonsäure.

Bevorzugte Untergrenzen für den Säuregehalt im Konditioniermittel sind 7 Gew.-%, 10 Gew.-%, 11 Gew.-%, 15 Gew.-%, 20 Gew.-%, 25 Gew.-%, 30 Gew.-%, 40 Gew.-% und 50 Gew.-%. Bevorzugte Obergrenzen sind 70 und 60 Gew.-%. Die genannten Ober- und Untergrenzen können beliebig zu erfindungsgemäßen Bereichen kombiniert werden. Weitere bevorzugte erfindungsgemäße Bereiche sind in Unteranspruch 2 angegeben.

Bevorzugte Bereiche für den pKs-Wert der verwendeten Säuren sind -6 bis 1, 1,5 oder 2, -5 bis 1, 1,5 oder 2 und -4 bis 1, 1,5 oder 2. Bevorzugt weist die erfindungsgemäße Zusammensetzung einen negativen pH-Wert auf, bevorzugt sind pH-Werte <-0,1, <-0,5 und <-1.

Die Auswahl hinreichend wenig flüchtiger Säuren erfolgt erfindungsgemäß anhand des Dampfdrucks der reinen Säure in einem Konditionierer, der bevorzugt bei 20°C 80 mbar, 50 mbar, 20 mbar, 10 mbar oder auch 5 mbar nicht übersteigt. Alternativ kann der Dampfdruck einer konzentrierten Säure bzw. einer gesättigten (bevorzugt wässrigen) Lösung der entsprechenden Säure betrachtet werden, der die genannten Grenzen bevorzugt nicht übersteigt. Bevorzugt werden erfindungsgemäß keine Halogenwasserstoffsäuren eingesetzt.

Der Konditionierer kann zusätzlich Verdickungsmittel wie beispielsweise partikuläre Füllstoffe oder höher viskose Stoffe enthalten. Geeignete Verdickungsmittel sind beispielsweise Kieselsäuren, Polyether oder Polyole sowie Polyacrylsäuren und deren Copolymere. Weitere Zusatzstoffe wie beispielsweise Farbstoffe oder grenzflächenaktive Stoffe (Tenside) können ebenfalls enthalten sein.

Gegenstand der Erfindung ist somit ein Kit zur Durchführung einer Infiltration von Zahnschmelz, der als Bestandteile eine Konditioniererzusammensetzung sowie einen Infiltranten aufweist. Bei dem Infiltranten handelt es sich bevorzugt um ein härtbares (beispielsweise lichthärtbares) Kunstharz, das nach der Vorbereitung der Läsion durch den Konditionierer in diese Läsion einpenetrieren und diese versiegeln kann.

Bevorzugt weist in diesem Kit der verwendete Infiltrant einen Penetrationskoeffizienten von mehr als 50 cm/s auf. Der Penetrationskoeffizient errechnet sich nach der nachfolgend aufgeführten, aus der sogenannten Washburn-Gleichung ableitbaren Gleichung:

$$PC = \left( \frac{\gamma \cdot \cos\theta}{2\eta} \right)$$

PC: Penetrationskoeffizient
$\gamma$: Oberflächenspannung des Infiltranten an der Grenzfläche zur Luft
$\theta$: Kontaktwinkel des Infiltranten an der Grenzfläche zur Schmelz
$\eta$: dynamische Viskosität des Infiltranten

Weitere Angaben zur Bestimmung des Penetrationskoeffizienten einschließlich Literaturhinweisen finden sich in der bereits genannten WO 2007/131725 A1.

Bevorzugt enthält der Infiltrant wenigstens ein Harz ausgewählt aus der Gruppe bestehend aus MMA, Methylmethacrylat; EMA, Ethylmethacrylat; n-BMA, n-Butylmethacrylat; IBMA, Isobutylmethacrylat, t-BMA, tert-Butylmethacrylat; EHMA, 2-Ethylhexylmethacrylat; LMA, Laurylmethacrylat; TDMA, Tridecylmethacrylat; SMA, Stearylmethacrylat; CHMA, Cyclohexylmethacrylat; BZMA, Benzylmethacrylat; IBXMA, Isobornylmethacrylate; MAA, Methacrylsäure; HEMA, 2-Hydroxyethylmethacrylat; HPMA, 2-Hydroxypropylmethacrylat; DMMA, Dimethylaminoethylmethacrylat; DEMA, Diethylaminoethylmethacrylat; GMA, Glycidylmethacrylat; THFMA, Tetrahydrofurfurylmethacrylat; AMA, Allylmethacrylat; EGDMA, Ethylenglycoldimethacrylat; 3EGDMA, Triethylenglycoldimethacrylat; 4EGDMA, Tetraethylenglycoldimethacrylat; BDMA, 1,3-Butylenglycoldimethacrylat; HDDMA, 1,6-Hexandioldimethacrylat; ETMA, Ethoxyethylmethacrylat; 3FM, Trifluorethylmethacrylat; 8FM, Octafluorpentylmethacrylat; AIB, Isobutylacrylat; TBA, tert-Butylacrylat; LA, Laurylacrylat; CEA, Cetylacrylat; STA, Stearylacrylat; CHA, Cyclohexylacrylat; BZA, Benzylacrylat; IBXA, Isobornylacrylat; 2-MTA, 2-Methoxyethylacrylat; ETA, 2-Ethoxyethylacrylat; EETA, Ethoxyethoxyethylacrylat; PEA, 2-Phenoxyethylacrylat; THFA, Tetrahydrofurfurylacrylat; HEA, 2-Hydroxyethylacrylat; HPA, 2-Hydroxypropylacrylat; 4HBA, 4-Hydroxybutylacrylat; DMA, Dimethylaminoethylacrylat; 1,4-Butylendioldiacrylat; 4EDA, Tetraethylenglycoldiacrylat; NDDA, 1,9- Nonandioldiacrylat; 3F, Trifluorethylacrylat; 17F, Heptadecafluorodecylacrylat; 2-PEA, 2-Phenoxyethylacrylat; TBCH, 4-tert-butylcyclohexylacrylat; DCPA, Dihydrodicyclopentadienylacrylat; EHA, 2-Ethylhexylacrylat; 3EGMA, Triethylenglycolmonomethacrylat; DEGDMA, Diethylenglycoldimethacrylat; PDDMA, 1,5-Pentandioldimethacrylat; BDDMA, 1,4-Butandioldimethacrylat; PRDMA, 1,3-Propandioldimethacrylat (alle vorstehenden Harze besitzen eine Viskosität < 30 mPas) oder aus der Gruppe bestehend aus DDDMA, 1,10-Decandioldimethacrylat; PEG400DA, Polyethylenglycol 400 Diacrylat, TMPTMA, Trimethylolpropantrimethacrylat, TMPTA, Trimethylolpropantriacrylat; DTMPTA; Di-Trimethylolpropantetraacrylat; DiPENTA, Di-Pentaerythritolpentaacrylat; PEG400DMA, Polyethylenglycol 400 Dimethacrylat, PEG300DA, Polyethylenglycol 300 Diacrylat, PEG300DMA, Polyethylenglycol 300 Dimethacrylat, BPA(EO)10DMA, Ethoxyliertes (10) Bisphenol-A-Dimethacrylat; BPA(EO)30DMA, Ethoxyliertes (30) Bisphenol-A-Dimethacrylat; PEG200DA, Polyethylenglycol 200 Diacrylat, PEG600DA, Polyethy-lenglycol 600 Diacrylat; NPG(PO)2DA Propoxyliertes (2) Neopentylglycol Diacrylat; BPA(EO)2DA, Ethoxyliertes (4) Bisphenol-A-Diacrylat; GPTA; propoxyliertes Glyceryltriacrylat; BPA(PO)2DMA, Propoxyliertes (2) Bisphenol-A-Dimethacrylat; DPEHA, Dipentaerythritolhexaacrylat; Bis-GMA, 2,2-bis[4-(2-Hydroxy-3-Methacryloxypropoxy)phenyl]propan; und UDMA, 1,6-bis(Methacryloxy-2-Ethoxycarbonylamino)-2,4,4-trimethylhexan; (alle vorstehenden Harze besitzen eine Viskosität > 30 mPas).

Weitere bevorzugte Untergruppen für die Auswahl des Infiltranten sind Triethylenglycoldimethacrylat und Trimethylolpropantrimethacrylat.

Der erfindungsgemäße Kit kann Applikationsstreifen, Reinigungsstreifen und/oder Trenneinrichtungen enthalten. Bei der Trenneinrichtung handelt es sich um ein Instrument, mit dem sich Approximalflächen zweier benachbarter Zähne geringfügig (beispielsweise ca. 300 μm) beabstanden lassen. Reinigungsstreifen können mit einer entsprechenden Reinigungslösung zur vorbereitenden Reinigung zu behandelnder Dentalflächen versehen sein. Applikationsstreifen können mit dem Konditioniermittel bzw. dem Infiltranten getränkt sein und so ein einfaches und präzises Applizieren dieser Bestandteile des Kits auch auf schwer zugängliche Approximalflächen von Zähnen ermöglichen.

In den nachfolgenden Ausführungsbeispielen und Vergleichsbeispielen wird die Eignung verschiedener Konditioniermittel zum Erodieren bzw. Demineralisieren von remineralisierten Oberflächenschichten natürlicher Schmelzläsionen an einem Modellsystem geprüft.

Dabei wurde die Ätzwirkung auf Hydroxylapatit [$Ca_5(PO_4)_3OH$]x2 (Hydroxylapatit-Plättchen) als Modellsystem für Schmelz, der zu etwa 95 % aus Hydroxylapatit besteht, untersucht. Unterschiedliche Konditioniermittel- oder Konditionierlösungen können hiermit unter reproduzierbaren Bedingungen bzgl. ihrer Ätzwirkung eingeschätzt und klassifiziert werden. Das Verfahren wird in der Publikation von A. Klocke et al., Dental Materials 19 (2003), 773 beschrieben.

Die Hydroxylapatit-Plättchen wurden durch hydraulisches Pressen (Einwaage: 300mg; Druck: 5000 bar; Zeit: 35min) von getrocknetem (2h, 60°C) Hydroxylapatit (Riedel de Haen) mit dem IR-Presswerkzeug (Firma LOT GmbH) hergestellt. Anschließend wurden die Presslinge so in einen Kunststoff (Paladur®, Heraeus Kulzer GmbH) eingebettet, dass nur eine Fläche unbedeckt blieb. Diese Fläche wurde mit Schleifpapier (Körnung: 500 und 1200) bearbeitet, um möglichst reproduzierbare Oberflächen zu erhalten und mit Pressluft vom Schleifstaub befreit. Der Durchmesser der Hydroxylapatitfläche betrug 13 mm.

Für die Ätzversuche wurden die Hydroxylapatit-Plättchen in 4 ml Konditionierlösung getaucht und im verschlossenen Probegefäß für 2 min auf einem Schüttelapparat (GFL; 100 min$^{-1}$) gerüttelt. Anschließend wurden die Hydroxylapatit-Plättchen vorsichtig mit einer Pinzette aus der Lösung genommen und mit Wasser (Qualität: Hyperpur) gründlich abgespült. Das Spülwasser wurde zurück in das Probegefäß gegeben. Der Inhalt des Probengefäßes wurde anschließend in einen 100 ml Meßkolben überführt, wobei das Probegefäß noch 2 mal mit Wasser ausgespült und das Waschwasser ebenfalls im Meßkolben aufgefangen wurde. Der Meßkolben wurde stets bis zur 100 ml-Eichmarke mit Wasser aufgefüllt. Mittels Atomabsorptionsspektroskopie (Perkin Elmer) wurde die Ca-Konzentration dieser Lösung bestimmt.

In der nachfolgenden Tabelle 1 sind erfindungsgemäße Zusammensetzungen als Konditioniermittel aufgeführt, die durch Verdünnen kommerziell erhältlicher Säuren in destilliertem Wasser mittels Magnetrührer hergestellt wurden. Die Säuren 1 bis 4 sind Vergleichsbeispiele, die Säuren 5 bis 9 erfindungsgemäße Beispiele. Die letzte Spalte der Tabelle 1 gibt die nach dem oben beschriebenen Versuch erhaltene Calciumkonzentration für die jeweilige Säure an.

Tabelle 1:

|  | Säure | pKs (reine Säure) | Gehalt [%] | Konz. [mol/l] | Ca-Konz. [mg/l] |
|---|---|---|---|---|---|
| 1. | HCl-Säure | -7 | 15 | 4,12 | 20,35 |
| 2. | HEDP* | 2-3 | 60 | 2,91 | 3,7 |
| 3. | Phosphorsäure | 2,2 | 43 | 4,39 | 0,8 |
| 4. | Citronensäure | 3,1 | 60 | 3,12 | 8,4 |
| 5. | Methansulfonsäure | -2 | 30 | 3,12 | 38,2 |
| 6. | Methansulfonsäure |  | 50 | 5,34 | 29,9 |
| 7. | p⁻ Toluolsulfonsäure | -2 | 50 | 2,90 | 34,1 |
| 8. | p-Toluolsulfonsäure |  | 30 | 1,74 | 42,3 |
| 9. | Trichloressigsäure | 0,08 | 50 | 3,06 | 33,3 |
| *1-Hydroxyethan-1,1-diphosphonsäure | | | | | |

Die Tabelle zeigt, dass die erfindungsgemäßen Zusammensetzungen selbst gegenüber 15%iger Salzsäure eine deutlich verbesserte Ätzwirkung aufweisen, ohne die beschriebenen Nachteile der Salzsäure aufzuweisen.

Zur Überprüfung der Lagerstabilität wurden aus den Zusammensetzungen 1, 5 und 8 Ätzgele hergestellt. Zu diesem Zweck wurden die Lösungen angedickt durch Zusatz von 5 Gew.-% pyrogener Kieselsäure (Aerosil® 200, Degussa) und 0,5 Gew.-% sorbitolbasiertes Polyetherpolyol mit einer Hydroxylzahl von 500. Die Vermischung erfolgt in einem Speedmixer (DAC 150 FV, Fa. Hauschild). Diese gelartigen Konditioniermittel wurden in übliche dentale Applikationsspritzen für Gele (Etching Gel, DMG) eingeführt und sieben Tage bei 60°C gelagert.

Zur Bestimmung der Lagerstabilität wurde die Säurekonzentration vor und nach der Lagerung gemessen. Die Ergebnisse sind in der nachfolgenden Tabelle 2 aufgeführt.

| Säure | Dampfdruck [mbar] | Gehalt [%] | Konz. vorh. | Konz. nachh. |
|---|---|---|---|---|
| HCl-Säure | 127 | 15 | 15,4 Gew.-% | 14,1 Gew.-% |
| Methansulfonsäure | <1 | 30 | Säurezahl 168,6 | Säurezahl 169,8 |
| p-Toluolsulfonsäure | 0,1 | 30 | Säurezahl 84,6 | Säurezahl 85,5 |

Man erkennt, dass die Säurekonzentration des Salzsäuregels durch Entweichen des HCl-Gases abgenommen hat, während die Konzentration der übrigen, kaum flüchtigen Säuren durch Verflüchtigung eines geringen Teils des als Lösungsmittel verwendeten Wasser geringfügig zugenommen hat.

Die bei den Ausführungsbeispielen verwendeten Säuren bilden unter den Bedingungen der Anwendung keine unlöslichen Calciumsalze. Dies ist generell ein bevorzugtes Merkmal der Erfindung.

**Patentansprüche**

1. Kit zur Durchführung einer Infiltration von Zahnschmelz, mit den Bestandteilen:

   a) einem Konditionierer für das Ätzen von Schmelzläsionen, der enthält:

   a2) 4 bis 80 Gew.-% wenigstens einer Säure, die einen pKs-Wert von 2 oder weniger aufweist;
   b2) ein protisches Lösungsmittel;
   wobei die Säure bei 20°C einen Sättigungsdampfdruck von 120 mbar oder weniger aufweist;

   b) einem Infiltranten.

2. Kit nach Anspruch 1, **dadurch gekennzeichnet, dass** der Säuregehalt des Konditionierers 11 bis 80 Gew.-%, vorzugsweise 15 bis 80 Gew.-%, weiter vorzugsweise 20 bis 70 Gew.-%, weiter vorzugsweise 25 bis 60 Gew.-% beträgt.

3. Kit nach Anspruch 1 oder 2, **dadurch gekennzeichnet, dass** der pKs-Wert der Säure -6 bis 1, vorzugsweise -5 bis 1, weiter vorzugsweise -4 bis 1 beträgt.

4. Kit nach einem der Ansprüche 1 bis 3, **dadurch gekennzeichnet, dass** das protische Lösungsmittel ausgewählt ist aus der Gruppe bestehend aus Wasser und Alkoholen.

5. Kit nach einem der Ansprüche 1 bis 4, **dadurch gekennzeichnet, dass** der Partialdampfdruck der Säure in der Zusammensetzung bei 20°C 80 mbar oder weniger, vorzugsweise 50 mbar oder weniger, weiter vorzugsweise 20 mbar oder weniger, weiter vorzugsweise 10 mbar oder weniger beträgt.

6. Kit nach einem der Ansprüche 1 bis 5, **dadurch gekennzeichnet, dass** der Konditionierer zusätzlich Verdickungsmittel enthält

7. Kit nach Anspruch 6, **dadurch gekennzeichnet, dass** die Verdickungsmittel ausgewählt sind aus der Gruppe bestehend aus partikulären Füllstoffen und Polyethern.

8. Kit nach einem der Ansprüche 1 bis 7, **dadurch gekennzeichnet, dass** der Infiltrant einen Penetrationskoeffizienten PK > 50 cm/s aufweist.

**9.** Kit nach Anspruch 8, **dadurch gekennzeichnet, dass** der Infiltrant wenigstens ein Harz enthält ausgewählt aus der Gruppe bestehend aus MMA, Methylmethacrylat; EMA, Ethylmethacrylat; n-BMA, n-Butylmethacrylat; IBMA, Isobutylmethacrylat, t-BMA, tert-Butylmethacrylat; EHMA, 2-Ethylhexylmethacrylat; LMA, Laurylmethacrylat; TDMA, Tridecylmethacrylat; SMA, Stearylmethacrylat; CHMA, Cyclohexylmethacrylat; BZMA, Benzylmethacrylat; IBXMA, Isobornylmethacrylate; MAA, Methacrylsäure; HEMA, 2-Hydroxyethylmethacrylat; HPMA, 2-Hydroxypropylmethacrylat; DMMA, Dimethylaminoethylmethacrylat; DEMA, Diethylaminoethylmethacrylat; GMA, Glycidylmethacrylat; THFMA, Tetrahydrofurfurylmethacrylat; AMA, Allylmethacrylat; EGDMA, Ethylenglycoldimethacrylat; 3EGDMA, Triethylenglycoldimethacrylat; 4EGDMA, Tetraethylenglycoldimethacrylat; BDMA, 1,3-Butylenglycoldimethacrylat; HDDMA, 1,6-Hexandioldimethacrylat; ETMA, Ethoxyethylmethacrylat; 3FM, Trifluorethylmethacrylat; 8FM, Octafluorpentylmethacrylat; AIB, Isobutylacrylat; TBA, tert-Butylacrylat; LA, Laurylacrylat; CEA, Cetylacrylat; STA, Stearylacrylat; CHA, Cyclohexylacrylat; BZA, Benzylacrylat; IBXA, Isobornylacrylat; 2-MTA, 2-Methoxyethylacrylat; ETA, 2-Ethoxyethylacrylat; EETA, Ethoxyethoxyethylacrylat; PEA, 2-Phenoxyethylacrylat; THFA, Tetrahydrofurfurylacrylat; HEA, 2-Hydroxyethylacrylat; HPA, 2-Hydroxypropylacrylat; 4HBA, 4-Hydroxybutylacrylat; DMA, Dimethylaminoethylacrylat; 1,4-Butylendioldiacrylat; 4EDA, Tetraethylenglycoldiacrylat; NDDA, 1,9- Nonandioldiacrylat; 3F, Trifluorethylacrylat; 17F, Heptadecafluorodecylacrylat; 2-PEA, 2-Phenoxyethylacrylat; TBCH, 4-tert-butylcyclohexylacrylat; DCPA, Dihydrodicyclopentadienylacrylat; EHA, 2-Ethylhexylacrylat; 3EGMA, Triethylenglycolmonomethacrylat; DEGDMA, Diethylenglycoldimethacrylat; PDDMA, 1,5-Pentandioldimethacrylat; BDDMA, 1,4-Butandioldimethacrylat; PRDMA, 1,3-Propandioldimethacrylat; DDDMA, 1,10-Decandioldimethacrylat; PEG400DA, Polyethylenglycol 400 Diacrylat, TMPTMA, Trimethylolpropantrimethacrylat, TMPTA, Trimethylolpropantriacrylat; DTMPTA; Di-Trimethylolpropantetraacrylat; DiPENTA, Di-Pentaerythritolpentaacrylat; PEG400DMA, Polyethylenglycol 400 Dimethacrylat, PEG300DA, Polyethylenglycol 300 Diacrylat, PEG300DMA, Polyethylenglycol 300 Dimethacrylat, BPA(EO)10DMA, Ethoxyliertes (10) Bisphenol-A-Dimethacrylat; BPA(EO)30DMA, Ethoxyliertes (30) Bisphenol-A-Dimethacrylat; PEG200DA, Polyethylenglycol 200 Diacrylat, PEG600DA, Polyethylenglycol 600 Diacrylat; NPG(PO)2DA Propoxyliertes (2) Neopentylglycol Diacrylat; BPA(EO)2DA, Ethoxyliertes (4) Bisphenol-A-Diacrylat; GPTA; propoxyliertes Glyceryltriacrylat; BPA(PO)2DMA, Propoxyliertes (2); Bisphenol-A-Dimethacrylat; DPEHA, Dipentaerythritolhexaacrylat; Bis-GMA, 2,2-bis[4-(2-Hydroxy-3-Methacryloxypropoxy)phenyl]propan; und UDMA, 1,6-bis(Methacryloxy-2-Ethoxycarbonylamino)-2,4,4-trimethylhexan.

**10.** Kit nach einem der Ansprüche 8 bis 9, **dadurch gekennzeichnet, dass** der Infiltrant wenigstens ein Harz enthält ausgewählt aus der Gruppe bestehend aus TEGDMA, Triethylenglycoldimethacrylat; und TMPTMA, Trimethylolpropantrimethacrylat.

**11.** Kit nach einem der Ansprüche 8 bis 10, **dadurch gekennzeichnet, dass** er wenigstens einen Applikationsstreifen und/oder Reinigungsstreifen und/oder eine Trenneinrichtung enthält.

**12.** Kit nach Anspruch 11, **dadurch gekennzeichnet, dass** er einen mit einem Konditionierer versehenen Applikationsstreifen enthält.

**13.** Kit nach Anspruch 11 oder 12, **dadurch gekennzeichnet, dass** er einen mit einem Infiltranten versehenen Applikationsstreifen enthält.

**Claims**

**1.** A kit for carrying out an infiltration of dental enamel, with the constituents:

a) a conditioner for etching enamel lesions which comprises

a1) 4 to 80% by weight of at least one acid which has a pKa of 2 or less;
a2) a protic solvent;
wherein the acid having a saturation vapor pressure at 20° C of 120 mbar or less, for use as conditioning agent for etching enamel lesions;

b) an infiltrant.

**2.** Kit according to claim 1, **characterised in that** the acid content of the conditioner is from 11 to 80% by weight, preferably 15 to 80% by weight, more preferably 20 to 70% by weight, more preferably 25 to 60% by weight.

3. Kit according to either claim 1 or claim2, **characterised in that** the pKa of the acid is from -6 to 1, preferably -5 to 1, more preferably -4 to 1.

4. Kit according to any of claims 1 to 3, **characterised in that** the protic solvent is selected from the group consisting of water and alcohols.

5. Kit according to any of claims 1 to 4, **characterised in that** the partial vapor pressure of the acid in the composition at 20° C is 80 mbar or less, preferably 50 mbar or less, more preferably 20 mbar or less, more preferably 10 mbar or less.

6. Kit according to any of claims 1 to 5, **characterised in that** the conditioner additionally comprises thickeners.

7. Kit according to claim 6, **characterised in that** the thickeners are selected from the group consisting of particulate fillers and polyethers.

8. Kit according to any of claims 1 to 7, **characterised in that** the infiltrant has a penetration coefficient PC of >50 cm/s.

9. Kit according to claim 8, **characterised in that** the infiltrant comprises at least one resin selected from the group consisting of MMA, methyl methacrylate; EMA, ethyl methacrylate; n-BMA, n-butyl methacrylate; IBMA, isobutyl methacrylate, t-BMA, tert-butyl methacrylate; EHMA, 2-ethylhexyl methacrylate; LMA, lauryl methacrylate; TDMA, tridecyl methacrylate; SMA, stearyl methacrylate; CHMA, cyclohexyl methacrylate; BZMA, benzyl methacrylate; IBXMA, isobornyl methacrylate; MAA, methacrylic acid; HEMA, 2-hydroxyethyl methacrylate; HPMA, 2-hydroxypropyl methacrylate; DMMA, dimethylaminoethyl methacrylate; DEMA, diethylaminoethyl methacrylate; GMA, glycidyl methacrylate; THFMA, tetrahydrofurfuryl methacrylate; AMA, allyl methacrylate; EGDMA, ethylene glycol dimethacrylate; 3EGDMA, triethylene glycol dimethacrylate; 4EGDMA, tetraethylene glycol dimethacrylate; BDMA, 1,3-butylene glycol dimethacrylate; HDDMA, 1,6-hexanediol dimethacrylate; ETMA, ethoxyethyl methacrylate; 3FM, trifluoroethyl methacrylate; 8FM, octafluoropentyl methacrylate; AIB, isobutyl acrylate; TBA, tert-butyl acrylate; LA, lauryl acrylate; CEA, cetyl acrylate; STA, stearyl acrylate; CHA, cyclohexyl acrylate; BZA, benzyl acrylate; IBXA, isobornyl acrylate; 2-MTA, 2-methoxyethyl acrylate; ETA, 2-ethoxyethyl acrylate; EETA, ethoxyethoxyethyl acrylate; PEA, 2-phenoxyethyl acrylate; THFA, tetrahydrofurfuryl acrylate; HEA, 2-hydroxyethyl acrylate; HPA, 2-hydroxypropyl acrylate; 4HBA, 4-hydroxybutyl acrylate; DMA, dimethylaminoethyl acrylate; 1,4-butylenediol diacrylate; 4EDA, tetraethylene glycol diacrylate; NDDA, 1,9-nonanediol diacrylate; 3F, trifluoroethyl acrylate; 17F, heptadecafluorodecyl acrylate; 2-PEA, 2-phenoxyethyl acrylate; TBCH, 4-tert-butylcyclohexyl acrylate; DCPA, dihydrodicyclopentadienyl acrylate; EHA, 2-ethylhexyl acrylate; 3EGMA, triethylene glycol monomethacrylate; DEGDMA, diethylene glycol dimethacrylate; PDDMA, 1,5-pentanediol dimethacrylate; BDDMA, 1,4-butanediol dimethacrylate; PRDMA, 1,3-propanediol dimethacrylate; DDDMA, 1,10-decanediol dimethacrylate; PEG400DA, polyethylene glycol 400 diacrylate, TMPTMA, trimethylolpropane trimethacrylate, TMPTA, trimethylolpropane triacrylate; DTMPTA; di-trimethylolpropane tetraacrylate; DiPENTA, di-pentaerythritol pentaacrylate; PEG400DMA, polyethylene glycol 400 dimethacrylate, PEG300DA, polyethylene glycol 300 diacrylate, PEG300DMA, polyethylene glycol 300 dimethacrylate, BPA(EO)10DMA, ethoxylated (10) bisphenol A dimethacrylate; BPA(EO)30DMA, ethoxylated (30) bisphenol A dimethacrylate; PEG200DA, polyethylene glycol 200 diacrylate, PEG600DA, polyethylene glycol 600 diacrylate; NPG(PO)2DA propoxylated (2) neopentyl glycol diacrylate; BPA(EO)2DA, ethoxylated (4) bisphenol A diacrylate; GPTA; propoxylated glyceryl triacrylate; DMTCDDA, dimethylol tricyclo[5.2.1.0<2,6>]decane dimethacrylate; BPA(PO)2DMA, propoxylated (2) bisphenol A dimethacrylate; DPEHA, dipentaerythritol hexaacrylate; bis-GMA, 2,2-bis[4-(2-hydroxy-3-methacryloxypropoxy)phenyl]propane; and UDMA, 1,6-bis(methacryloxy-2-ethoxycarbonylamino)-2,4,4-trimethylhexane.

10. Kit according to any of claims 8 to 9, **characterised in that** the infiltrant comprises at least one resin selected from the group consisting of TEGDMA, triethylene glycol dimethacrylate; and TMPTMA, trimethylolpropane trimethacrylate.

11. Kit according to any of claims 8 to 10, **characterised in that** it comprises at least one application strip and/or cleaning strip and/or a separator.

12. Kit according to claim 11, **characterised in that** it comprises an application strip provided with a conditioner.

13. Kit according to either claim 11 or claim 12, **characterised in that** it comprises an application strip provided with an infiltrant.

## Revendications

1. Trousse pour la mise en oeuvre d'une infiltration d'émail dentaire, comprenant les constituants suivants :

 a) un agent de conditionnement pour le décapage de lésions de l'émail, qui contient :

 a2) 4 à 80 % en poids d'au moins un acide qui présente un pKs de 2 ou moins ;
 b2) un solvant protique ; et
 l'acide présentant à 20°C une pression de vapeur saturante de 120 mbar ou moins ;

 b) un agent d'infiltration.

2. Trousse selon la revendication 1, **caractérisée en ce que** la teneur en acide de l'agent de conditionnement est de 11 à 80 % en poids, de préférence de 15 à 80 % en poids, plus préférentiellement de 20 à 70 % en poids, plus préférentiellement de 25 à 60 % en poids.

3. Trousse selon la revendication 1 ou 2, **caractérisée en ce que** le pKs de l'acide est de -6 à 1, de préférence de -5 à 1, plus préférentiellement de -4 à 1.

4. Trousse selon l'une des revendications 1 à 3, **caractérisée en ce que** le solvant protique est choisi dans le groupe consistant en l'eau et les alcools.

5. Trousse selon l'une des revendications 1 à 4, **caractérisée en ce que** la pression partielle de vapeur de l'acide dans la composition à 20°C est de 80 mbar ou moins, de préférence de 50 mbar ou moins, plus préférentiellement de 20 mbar ou moins, plus préférentiellement de 10 mbar ou moins.

6. Trousse selon l'une des revendications 1 à 5, **caractérisée en ce que** l'agent de conditionnement contient en outre des épaississants.

7. Trousse selon la revendication 6, **caractérisée en ce que** les épaississants sont choisis dans le groupe consistant en les matières de charge particulaires et les polyéthers.

8. Trousse selon l'une des revendications 1 à 7, **caractérisée en ce que** l'agent d'infiltration présente un coefficient de pénétration PK > 50 cm/s.

9. Trousse selon la revendication 8, **caractérisée en ce que** l'agent d'infiltration contient au moins une résine choisie dans le groupe consistant en le MMA, méthacrylate de méthyle ; l'EMA, méthacrylate d'éthyle ; le n-BMA, méthacrylate de n-butyle ; l'IBMA, méthacrylate d'isobutyle ; le t-BMA, méthacrylate de tert-butyle ; l'EHMA, méthacrylate de 2-éthylhexyle ; le LMA, méthacrylate de lauryle ; le TDMA, méthacrylate de tridécyle ; le SMA, méthacrylate de stéaryle ; le CHMA, méthacrylate de cyclohexyle ; le BZMA, méthacrylate de benzyle ; l'IBXMA, méthacrylate d'isobornyle ; le MAA, acide méthacrylique ; le HEMA, méthacrylate de 2-hydroxyéthyle ; le HPMA, méthacrylate de 2-hydroxypropyle ; le DMMA, méthacrylate de diméthylaminoéthyle ; le DEMA, méthacrylate de diéthylaminoéthyle ; le GMA, méthacrylate de glycidyle ; le THFMA, méthacrylate de tétrahydrofurfuryle ; l'AMA, méthacrylate d'allyle ; l'EGDMA, diméthacrylate d'éthylèneglycol ; le 3EGDMA, diméthacrylate de triéthylèneglycol ; le 4EGDMA, diméthacrylate de tétraéthylèneglycol ; le BDMA, dimethacylate de 1,3-butylèneglycol ; le HDDMA, diméthacrylate de 1,6-hexanediol ; l'ETMA, méthacrylate d'éthoxyéthyle ; le 3FM, méthacrylate de trifluoréthyle ; le 8FM, méthacrylate d'octafluoropentyle ; l'AIB, acrylate d'isobutyle ; le TBA, acrylate de tert-butyle ; le LA, acrylate de lauryle ; le CEA, acrylate de cétyle ; le STA, acrylate de stéaryle ; le CHA, acrylate de cyclohexyle ; le BZA, acrylate de benzyle ; l'IBXA, acrylate d'isobornyle ; le 2-MTA, acrylate de 2-méthoxyéthyle ; l'ETA, acrylate de 2-éthoxyéthyle ; l'EETA, acrylate d'éthoxyéthoxyéthyle ; le PEA, acrylate de 2-phénoxyéthyle ; le THFA, acrylate de tétrahydrofurfuryle ; le HEA, acrylate de 2-hydroxyéthyle ; le HPA, acrylate de 2-hydroxypropyle ; le 4HBA, acrylate de 4-hydroxybutyle ; le DMA, acrylate de diméthylaminoéthyle ; le diacrylate de 1,4-butylènediol ; le 4EDA, diacrylate de tétraéthylèneglycol ; le NDDA, diacrylate de 1,9-nonanediol ; le 3F, acrylate de trifluoréthyle ; le 17F, acrylate d'heptadécafluorodécyle ; le 2-PEA, acrylate de 2-phénoxyéthyle ; le TBCH, acrylate de 4-tert-butylcyclohexyle ; le DCPA, acrylate de dihydrodicyclopentadiényle ; l'EHA, acrylate de 2-éthylhexyle ; le 3EGMA, monométhacrylate de triéthylèneglycol ; le DEGDMA, diméthacrylate de diéthylèneglycol ; le PDDMA, diméthacrylate de 1,5-pentanediol ; le BDDMA, diméthacrylate de 1,4-butanediol ; le PRDMA, diméthacrylate de 1,3-propanediol ; le DDDMA, diméthacrylate de 1,10-décanediol ; le PEG400DA, diacrylate de polyéthylèneglycol 400 ; le TMPTMA, triméthacrylate de triméthylolpropane ; le TMPTA, triacrylate de triméthylolpropane ; le DTMPTA, tétraacrylate de di-triméthylolpropane ; le DiPENTA, pentacrylate de dipentaérythritol ; le PEG400DMA, diméthacrylate de polyéthylèneglycol 400 ; le PEG300DA, diacrylate de polyéthylèneglycol 300 ; le PEG300DMA, diméthacrylate de polyéthylèneglycol 300 ; le BPA(EO)10DMA, diméthacrylate de bisphénol-A à 10 moles d'oxyde d'éthylène ; le BPA(EO)30DMA, diméthacrylate de bisphénol-A à 30 moles d'oxyde d'éthylène ; le PEG200DA, diacrylate de polyéthylèneglycol 200 ; le PEG600DA, diacrylate de polyéthylèneglycol 600 ; le NPG(PO)2DA, diacrylate de néopentylglycol à 2 moles d'oxyde de propylène ; le BPA(EO)2DA, diacrylate de bisphénol-A à 4 moles d'oxyde d'éthylène ; le GPTA, triacrylate de glycéryl propoxylé ; le BPA(PO)2DMA, diméthacrylate de bisphénol-A à 2 modes d'oxyde de propylène ; le DPEHA, hexaacrylate de dipentaérythritol ; le bis-GMA, 2,2-bis[4-(2-hydroxy-3-méthacryloxypropoxy)phényl]propane ; et l'UDMA, 1,6-bis(méthacryloxy-2-éthoxycarbonylamino)-2,4,4-triméthylhexane.

10. Trousse selon l'une des revendications 8 à 9, **caractérisée en ce que** l'agent d'infiltration contient au moins une résine choisie dans le groupe consistant en le TEGDMA, diméthacrylate de triéthylèneglycol ; et le TMPTMA, triméthacrylate de triméthylolpropane.

11. Trousse selon l'une des revendications 8 à 10, **caractérisée en ce qu'**elle contient au moins une bandelette d'application et/ou une bandelette de nettoyage et/ou un dispositif séparateur.

12. Trousse selon la revendication 11, **caractérisée en ce qu'**elle contient une bandelette d'application pourvue d'un agent de conditionnement.

13. Trousse selon la revendication 11 ou 12, **caractérisée en ce qu'**elle contient une bandelette d'application pourvue d'un agent d'infiltration.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007131725 A1

**In der Beschreibung aufgeführte Nicht-Patentliteratur**

- **A. KLOCKE et al.** *Dental Materials,* 2003, vol. 19, 773